# EUROPÄISCHE PATENTANMELDUNG

(11) **EP 0 667 355 A1**
(43) Veröffentlichungstag der Anmeldung: **16.08.1995**
(21) Anmeldenummer: 95101554.4
(22) Anmeldetag: 06.02.1995
(51) Int. Cl.: C07K 14/815, A61K 47/48

(54) **Hirudinderivate und Verfahren zu deren Herstellung**

(30) Priorität: 10.02.1994 DE 4404168
(71) Anmelder: HOECHST AKTIENGESELLSCHAFT, 65929 Frankfurt am Main (DE)
(72) Erfinder: Obermeier, Rainer, Dr., D-65795 Hattersheim (DE); Ludwig, Jürgen, D-63636 Brachttal (DE); Tripier, Dominique, Dr., D-65817 Eppstein (DE); Hropot, Max, Dr., D-65439 Flörsheim (DE)

(57) **Zusammenfassung**

Hirudinderivate sind herstellbar durch selektiven enzymatischen Austausch eines Molekülteils durch ein Aminoderivat. Die Hirudinderivate eignen sich zur Herstellung von Arzneimitteln mit gerinnungshemmender Wirkung.

## Beschreibung

Die Erfindung betrifft Hirudinderivate, deren Herstellung durch selektiven enzymatischen Austausch eines Molekülteiles durch ein Aminderivat und die Verwendung der Hirudinderivate als Arzneimittel.

Hirudin ist ein aus 65 Aminosäureresten bestehendes Protein, das aus den Speicheldrüsen des Blutegels (Hirudo medicinalis) isoliert werden kann. Als selektiver Thrombininhibitor wirkt es gerinnungshemmend (Pharmazie 36, 633-660 (1981)). Das einkettige Polypeptid besitzt drei Disulfidbrücken, die die Domäne zwischen den Aminosäuren der Position 1 bis 49 als eine stark gefaltete, kompakte Proteintertiärstruktur stabilisieren. Die C-terminale Aminosäuresequenz 55 bis 65 stellt sich linearisiert als Ankersequenz zur Bindung an das Thrombin dar. Natives Hirudin trägt einen Sulfatrest an der phenolischen OH-Gruppe des Tyrosinrestes in Position 63, was den ohnehin durch Cluster von sauren Aminosäureresten verursachten aciden Charakter dieser Sequenzregion verstärkt. Abspaltung der Sulfatgruppe erhöht den Wert der Dissoziationskonstanten K_{D}, der für die Wechselwirkung Hirudin/Thrombin in einem Bereich von 60 pmol-20 fmol angegeben wird.

Chemisch und enzymatisch hergestellte Hirudinderivate sind in EP 0 493 588 (Nitro-Gruppe, Halogensubstitution) und DE 4 224 213 (C-terminale, enzymatische Amidierung) zu finden. Konjugate von freien Aminogruppen des N-Terminus oder der Lysyl-Seitenketten mit Sacchariden oder Polyethylenglykol (PEG) werden in US 4 179 337 und US 4 847 325 gezeigt.

Zur Verwendung als langwirkende Arzneimittel werden herkömmliche HirudinPolyethylenglykol-Derivate hergestellt, in dem eine funktionelle Gruppe einer im Hirudin enthaltenen Aminosäure mit einem Polyethylenglykolrest (PEG) chemisch gekoppelt wird (vgl. EP 0 345 616). Es entstehen dabei keine definierten Verbindungen, da der Rest in der Regel an mehreren funktionellen Stellen des Hirudin-Moleküls gebunden werden kann.

Hirudin enthält die Aminosäuren Lysin an drei, Tyrosin an zwei Positionen und Phenylalanin an einer Position im Molekül. Solche Stellen in einem Protein werden gewöhnlich von Serinproteasen wie Trypsin oder Chymotrypsin gespalten. Bisher bekannte enzymatische Semisynthese-Verfahren (The Peptides, S. Udenfriend und J. Meienhofer (Eds.), Vol. 9, Acad. Press, NY. 1987, 103-165) schienen daher nicht auf Hirudin übertragbar zu sein. Nach dem Fachwissen ist die bevorzugte Bildung von inaktiven Abbauprodukten des Hirudins zu erwarten.

Es wurde gefunden, daß unter bestimmten Reaktionsbedingungen eine selektive Transpeptidierung bei enzymatischen Semisynthesereaktionen von Hirudin mit Chymotrypsin, Trypsin oder einem vergleichbaren Enzym (z.B. Lysylendopeptidase) erfolgt, die zu neuen Verbindungen führt.

Gegenstand der Erfindung sind Verbindungen der Formel oder II:
AO-A1-A2-(Hirudin 3-36)-(Y), (Hirudin 37-65) (I),
AO-A1-A2-(Hirudin 3-63)-(Y) (11),
   dabei ist Y ein Aminderivat,
   A1 und A2 sind unabhängig voneinander ein Aminosäurerest, und
   AO ist ein Aminosäurerest oder Wasserstoffatom.

Die Nomenklatur der Formeln I und II bezieht sich auf die Veröffentlichung von D. Tripier in Folia Haematol. (Leipzig) 115, 30-35 (1988).

Das Komma in Formel I bedeutet, daß die Aminosäuresequenz des zugrundeliegenden Hirudins zwischen den Positionen 36 und 37 unterbrochen ist und die Verbindung zwei Hirudin-Fragmente enthält, die über Disulfidbrücken verbunden sind. Das Aminderivat Y ist durch eine Amidbindung mit der Aminosäure in Position 36 der ursprünglichen Sequenz des zugrundeliegenden Hirudins verknüpft.

Formel II zeigt eine Verbindung, die ein Aminderivat Y enthält, das durch eine Amidbindung mit der Aminosäure in Position 63 der ursprünglichen Sequenz des zugrundeliegenden Hirudins verknüpft ist.

Aminderivate Y sind bevorzugt Verbindungen der Formel Illa oder Illb:
H₂ N-R-X (Illa),
A-R¹⁻X (Illb),
dabei ist:
   A
      a) ein Aminosäurerest,
      b) ein Peptid mit 2 bis 10 Aminosäureresten,
   R
      a) (C₁-C₁₀)-Alkyl, verzweigt oder geradkettig,
      b) (C₁-C₁₀)-Alkyl, verzweigt oder geradkettig, ein- oder mehrfach unabhängig voneinander substituiert durch
         1) Phenyl,
         2) Indolyl,
         3) Imidazolyl oder
         4) Phenyl, ein- oder mehrfach substituiert durch Hydroxyl,
      c) Phenyl oder
      d) Naphthyl,
   R
      a) Wasserstoffatom,
      b) kovalente Bindung,
      c) Zucker wie Glucose, Fructose, Mannose, Galactose, Ribose, Ribulose oder Xylose,
      d) Polysaccharid, enthaltend 2 bis 10 Zucker, oder
      e) -[O-(CH₂)ₘ]ₙ-, dabei ist: m eine ganze Zahl 2, 3, 4 oder 5 und n eine ganze Zahl von 1 bis 100, und
   X
      a) Wasserstoffatom,
      b) -O_{R2},
      c) -S_{R2},
      d) -NH_{R}2,
      e) -COOR² oder
      f) A, dabei ist: R²
         1) Wasserstoffatom,
         2) (C₁-C₁₀)-Alkyl, verzweigt oder geradkettig,
         3) (C₁-C₁₀)-Alkyl, verzweigt oder geradkettig, ein-oder mehrfach substituiert durch
            3.1 Phenyl,
            3.2 Indolyl,
            3.3 Imidazolyl oder
            3.4 Phenyl, ein- oder mehrfach substituiert durch Hydroxyl,
         4) Phenyl oder
         5) Naphthyl.

Bevorzugt sind Aminderivate Y der Formel Illa oder Illb, dabei ist:
A ein Peptid mit 2 bis 5 Aminosäureresten, R Ethyl, substituiert durch
   1) Phenyl,
   2) Indolyl,
   3) Imidazolyl oder
   4) 4-Hydroxyphenyl,
R -[O-(CH₂)ₘ]ₙ-, dabei ist:
   m die Zahl 2 und
   n eine ganze Zahl von 20 bis 50,
X
   a) Wasserstoffatom,
   b) -O_{R2},
   c) -NHR² oder
   d) -COO_{R}², dabei ist:

   R²
      1) Wasserstoffatom,
      2) (C₁-C₅)-Alkyl oder
      3) Phenyl.

Insbesondere bevorzugt sind Aminderivate Y der Formel Illa oder Illb, dabei ist:
A ein Peptid mit 2 bis 5 Aminosäureresten aus der Gruppe Thr oder Arg,
R
   a) Wasserstoffatom,
   b) kovalente Bindung,
   c) Glucose oder
   d) Polyethylenglykol mit einem Molekulargewicht von 100 bis 3000 g/mol,
X
   a) Wasserstoffatom oder
   b) -O_{R}2,
   dabei ist R² tertiär Butyl.

Insbesondere bevorzugt sind -[0-(CH₂)_{m]n}-Reste mit einem Molekulargewicht von 1500 g/mol.

Unter einer Aminosäure wird eine natürliche, eine genetisch codierbare, nicht natürliche, nicht codierbare, L-oder D-Aminosäure verstanden.

Beispiele einer L- oder D-Aminosäure sind: Alanin, Valin, Leucin, Isoleucin, Serin, Threonin, Cystein, Cystin, Methionin, Ornithin, Citrullin, Arginin, Lysin, Asparagin, Asparaginsäure, Glutaminsäure, Glutamin, Phenylalanin, Tyrosin, Thyroxin, Prolin, Hydroxyprolin, Tryptophan, Histidin. Glycin und gamma-Aminobuttersäure sind weitere Beispiele für eine Aminosäure.

Weiterer Gegenstand der Erfindung ist ein Verfahren zur Herstellung von Verbindungen der Formel I und 11, das dadurch gekennzeichnet ist, daß Aminderivate und Hirudin in Gegenwart von Proteasen, vorzugsweise Serinproteasen, bevorzugt Chymotrypsin, Trypsin, trypsinähnlicher Enzyme oder Lysylendopeptidase, umgesetzt werden.

Hirudine können beispielsweise eingesetzt werden, die bekannt sind aus:
EP 142 860, EP 158 564, EP 158 986, EP 168 342, EP 171 024, EP 193 175, EP 209 061, EP 227 938, DE 34 45 517, DE 38 05 540.6; Chang, FEBS 164, 307 (1983); Tripier (Folia Haematol. (Leipzig) 115, 30-35 (1988). Das Sequenzprotokoll ID No: 1 zeigt exemplarisch die Struktur eines Hirudins.

Als Hirudine kommen beispielsweise die in oben zitierten Literaturstellen beschriebenen Verbindungen in Betracht, insbesondere die in EP 171 024, EP 158 986, EP 209 061 und DE 38 05 540.6 beschriebenen Verbindungen, z.B.:
Leu-Thr-Tyr-Thr-Asp-Cys-Thr-Glu-Ser-Gly-Gln-Asn-Leu-Cys-1 5 10
Leu-Cys-Glu-Gly-Ser-Asn-Val-Cys-Gly-Gln-Gly-Asn-Lys-Cys-15 20 25
Ile-Leu-Gly-Ser-Asp-Gly-Glu-Lys-Asn-Gln-Cys-Val-Thr-Gly-30 35 40
Glu-Gly-Thr-Pro-Lys-Pro-Gln-Ser-His-Asn-Asp-Gly-Asp-Phe-45 50 55
Glu-Glu-Ile-Pro-Glu-Glu-Tyr-Leu-Gln. 60 65

Diese Hirudine können nach dem Fachmann allgemein bekannten Methoden der Peptidchemie oder durch äquivalente bekannte Methoden hergestellt werden. Alternativ sind die genannten Hirudine auch durch dem Fachmann bekannte gentechnische Methoden zugänglich.

Verbindungen der Formel werden aus einem Hirudin und einem im Überschuß vorliegenden, geeigneten Aminderivat der Formeln Illa oder Illb mit Hilfe von Trypsin oder einem Trypsin-ähnlichen Enzym, z.B. Lysylendopeptidase, gebildet. Geeignete Aminderivate sind solche, die im Falle des Einsatzes von Trypsin keine freie (d.h. keine ungeschützte) Arginyl- und Lysyl-Seitenkette oder bei Verwendung von Lysylendopeptidase keine freie Lysyl-Seitenkette tragen. Es erfolgt eine Transpeptidierung am C-Terminus des Lysinrestes in Position 36 der Aminosäuresequenz des eingesetzten Hirudins. Dabei wird die Peptidbindung-Lys-Asn- (36 - 37) geöffnet und das Aminderivat eindeutig an die Carboxylgruppe des Lys-Restes gebunden. Dadurch entsteht ein "zweikettiges" Hirudin-Derivat, welches von drei Disulfidbrücken zusammengehalten wird. So lassen sich mittels N-terminaler Aminosäure-Sequenzanalyse (Edman-Abbau) zwei endständige Aminosäurereste AO bzw. A1, wenn AO entfällt, und Asn37 (Asparagin in Position 37 der zugrundeliegenden Hirudin-Aminosäuresequenz) nachweisen, was eine zweikettige Struktur des Hirudin-Derivates belegt.

Verbindungen der Formel II werden aus einem Hirudin und einem im Überschuß vorliegenden, geeigneten Aminderivat der Formeln Illa oder Illb mit Hilfe von Chymotrypsin oder einem vergleichbar wirkenden Enzym gebildet. Geeignete Aminderivate sind hier solche, die keine freie Tyrosyl-Seitenkette tragen. Es erfolgt eine Transpeptidierung am C-Terminus des Tyrosinrestes in Position 63 der Aminosäuresequenz des eingesetzten Hirudins. Dabei wird das Dipeptid, bestehend aus den Aminosäuren der Positionen 64 und 65 der Aminosäuresequenz des zugrundeliegenden Hirudins, durch ein Aminderivat ausgetauscht.

Das Aminderivat wird im Überschuß eingesetzt. Bevorzugt ist ein 50- bis 150-facher molarer Überschuß des Aminderivats zur Hirudinmenge. Das Aminderivat wird bevorzugt in möglichst hoher Konzentration eingesetzt. Als Lösemittel dienen Wasser oder ein Gemisch von Wasser und einem nicht protonierenden organischen Lösemittel wie Dimethylformamid. Das Gemisch enthält vorzugsweise weniger als 25 Vol.-% (v/v) Wasser. Der Einsatz von mit Wasser mischbaren organischen Lösemitteln ist im Falle von schwer wasserlöslichen Aminderivaten von Vorteil. Auf diese Weise kann die Homogenität des Reaktionsgemisches hergestellt werden. Die Enzymmenge entspricht vorzugsweise 5 bis 10 Gew.-% der eingesetzten Hirudinmenge. Die Reaktion erfolgt bevorzugt bei einem pH-Wert von 4 bis 6 und bei einer Reaktionstemperatur von 0 bis 40 °C, besonders bevorzugt bei einer Temperatur von 0 bis 10 C.

Die verwendeten Enzyme können unterschiedliche biologische Herkunft haben, z.B. kann Trypsin vom Schwein oder Rind stammen.

Die enzym-katalysierte Umsetzung von Hirudin führt bei Verwendung von Aminosäure- oder Oligopeptidestern als Aminderivat zu den entsprechenden Hirudinderivat-Estern. Falls gewünscht, können die Estergruppen durch übliche Methoden abgespalten werden. Vorteilhaft ist, die Estergruppe der Zwischenprodukte jedoch erst nach einer chromatographischen Reinigung abzuspalten.

Die enzym-katalysierte Semisynthese von Hirudinderivaten gemäß der Erfindung ist ein Verfahren, das gentechnische Verfahren zur Änderung der Hirudin-Struktur komplementiert. Insbesondere können mit Hilfe der Semisynthese nicht codierbare Aminosäuren wie D-Aminosäuren oder beliebige Aminderivate gezielt eingeführt werden.

Das Verfahren, das Gegenstand der Erfindung ist, ermöglicht zum Beispiel die Herstellung eines definierten Hirudin-Polyethylenglykol-Derivates der Formel 1 oder 11, das als Depotpräparat medizinisch genutzt werden kann. Ein Polyethylenglykol, das mit einer Aminosäure oder einem Oligopeptid verestert ist, kann als Aminderivat sehr selektiv mit einem Hirudin umgesetzt werden, so daß der Polyethylenglykolrest nur einmal an einer definierten Position gebunden ist.

Andere gemäß der Erfindung hergestellte Hirudin-Derivate können zur transdermalen Applikation, z.B. lontophorese, von therapeutischem Nutzen sein. Die Verabreichung von Hirudin-Derivaten durch lontophorese ist weiterer Gegenstand der Erfindung.

Ein weiterer Gegenstand der Erfindung sind Arzneimittel, die eine wirksame Menge von mindestens einer Verbindung der Formel I und/oder II enthalten.

Gegenstand der Erfindung ist ferner die Verwendung der Verbindungen der Formel I und II als Arzneimittel mit gerinnungshemmender Wirkung.

Die nachfolgenden Beispiele, in denen gentechnisch gewonnenes DesulfatoTyr63-Hirudin (Sequenzprotokoll ID No: 1) verwendet wird, sollen die Erfindung erläutern, jedoch ohne auf diese einzuschränken.

### Beispiel 1: Herstellung von Hirudin

Die Synthese von Hirudin erfolgt beispielsweise in Hefezellen, die Hirudin gemäß EP 0324712 ausschleusen. Hirudin wird anschließend über eine Säule mit ΘDiaion HP20 (Mitsubishi Chem. Ind., Japan) chromatographisch angereichert (siehe EP 0316650). Nach Dialyse und anschließender Affinitätschromatographie über Thrombin-Sepharose erfolgt die endgültige Reinigung über "reversed phase" HPLC.

### Beispiel 2:

3 g gentechnisch gewonnenes Desulfato-Tyr63-Hirudin (Sequenzprotokoll SEQ ID NO: 1) werden zusammen mit 20 g Thr(But)OBut-Acetat (mit tert. Butyl-Schutzgruppen versehenes Threonin; zur Herstellung siehe EP 0056951) in 6 ml Wasser gelöst und mit 190 mg Chymotrypsin (Merck, Darmstadt) versetzt. Der pH-Wert der Lösung wird auf 4,5 eingestellt und die Reaktionsmischung 72 Stunden bei 4°C gelagert.

Danach wird die Reaktionsmischung mit 100 ml Methanol verdünnt und 300 ml Dimethylether zugegeben. Der gewonnene Niederschlag wird abzentrifugiert. Der Rückstand wird erneut mit einem Gemisch aus Methanol/Diethylether (1:2 v/v) gewaschen, abzentrifugiert mit Ether gewaschen und unter vermindertem Druck getrocknet. Ausbeute: 3,6 g Rohprodukt.

Das Rohprodukt besteht aus nicht umgesetztem Hirudin, dem gewünschten Hirudin-Derivat und durch enzymatische Spaltung entstandene Nebenprodukte. Die Analyse des Rohproduktes mittels analytischer HPLC ergab: 39,6 % (Hirudin1-63)-Thr(But)OBut(64), 45,9 % (Hirudin 1-65), 6,3 % Spaltprodukte.

Das Rohprodukt wird auf einer präparativen HPLC-Säule (5 x 25 cm, stationäre Phase ist ein reverse Phase C,8-Material) durch Gradientenelution mit Acetonitril/Wasser/Trifluoressigsäure chromatographisch getrennt. (Hirudin 1-63)-Thr(But)OBut eluiert wegen der lipophilen tert. Butyl-Schutzgruppen mit deutlich verzögerter Retentionszeit. Die das reine Zwischenprodukt enthaltende Fraktion wird gesammelt, das Acetonitril wird unter vermindertem Druck abgedampft und die wäßrige Lösung gefriergetrocknet. Ausbeute: 963 mg (Hirudin-63)-Thr(But)OBut(64) (Reinheit: 93,6 %).

Das erhaltene Hirudin-Derivat wird in 20 ml 95%iger Trifluoressigsäure gelöst und 45 Minuten bei Raumtemperatur gehalten. Danach wird mit 100 ml eisgekühltem Dimethylether versetzt. Der erhaltene Niederschlag wird abzentrifugiert, weitere dreimal mit Ether gewaschen und anschließend unter vermindertem Druck getrocknet.

Ausbeute: 802 mg (Hirudin-63)-Thr64 (siehe Sequenzprotokoll SEQ ID NO:2).

Die Aminosäureanalyse des Produktes entspricht der nach der Aminosäuresequenz zu erwartenden Zusammensetzung.

### Beispiel 3:

3 g gentechnisch gewonnenes Desulfato-Tyr63-Hirudin (Sequenzprotokoll ID-No. 1) werden wie in Beispiel 2 mit 41 g Thr(But)-Arg-Arg (synthetisiert nach Standardverfahren) und 170 mg Chymotrypsin (Merck, Darmstadt) umgesetzt.

Nach beendigter Reaktion wird die Mischung mit 300 ml Methanol und 150 ml Dimethylether versetzt. Der abzentrifugierte Niederschlag wird dreimal mit Methanol/Ether und anschließend mit Ether gewaschen und im Vakuum getrocknet. Ausbeute: 4,8 g Rohprodukt. Die analytische HPLC (stationäre Phase C↑ε-Kieselgel, Laufmittel Acetonitril/H₂0/Trifluoressigsäure) ergibt folgende Zusammensetzung: 65,2 % (Hirudin 1-63)-Thr(But) (64)-Arg(65)-Arg(66)-OH; 3,9 % (Hirudin-65); 18,7 % Spaltprodukte. Chromatographische Reinigung und Isolierung des Produkts erfolgt wie in Beispiel 2.

Ausbeute: 1,55 g (Hirudin1-63)-Thr(But) (64)-Arg(65)-Arg(66)-OH;

Reinheit: 90,3 %.

Die Abspaltung der tert. Butyl-Schutzgruppe der Threoninseitenkette erfolgt in bekannter Weise wie in Beispiel 2. Ausbeute: 1,2 g (Hirudinl-63)-Thr(64)-Arg(65)-Arg(66)-OH (siehe Sequenzprotokoll SEQ ID NO:3).

Die Aminosäureanalyse des Produktes entspricht der nach der Aminosäuresequenz zu erwartenden Zusammensetzung.

Bei der Bestimmung der anticoagulatorischen Wirkung nach intravenöser Gabe am Rhesus-Affen (1 mg/kg) zeigt dieses Hirudin-Derivat trotz Änderungen des isoelektrischen Punktes des Hirudin-Derivates eine nahezu gleiche Wirksamkeit wie das Ausgangs-Hirudin (Testbedingungen siehe: J. Jürgens, Dtsch. Arch. Klin. Med. 200, 67 (1952)).

### Beispiel 4:

3 g gentechnisch gewonnenes Desulfato-Tyr63-Hirudin (Sequenzprotokoll SEQ ID NO: 1) werden wie in Beispiel 2 mit 20 g Thr(But)OBut-Acetat und 200 mg Trypsin (Merck, Darmstadt) umgesetzt.

Nach 72 Stunden wird die Reaktion abgebrochen und wie in Beispiel 2 aufgearbeitet. Ausbeute: 4,2 g Rohprodukt mit folgender Zusammensetzung: 55 % (Hirudin 1-36)-Thr(But)OBut, (Hirudin37-65); 31 % nicht umgesetztes Hirudin; 15 % Spaltprodukte. Die chromatographische Reinigung und anschließende Isolierung erfolgt wie in Beispiel 2. Ausbeute: 1,65 g (Hirudin 1-36)-Thr(But)OBut, (Hirudin37-65).

Die Abspaltung der tert-Butylschutzgruppen des Threoninrestes erfolgt wie in Beispiel 2. Ausbeute: 1,3 g (Hirudin 1-36)-Thr, (Hirudin37-65). Die Struktur der ersten Kette des Hirudin-Derivates, (Hirudin1-36)-Thr, wird durch SEQ ID NO:4 wiedergegeben, die Struktur der zweiten Kette des Hirudin-Derivates, (Hirudin37-65), durch Sequenzprotokoll SED ID NO:5.

Die Aminosäureanalyse des Produktes entspricht der nach der Aminosäuresequenz zu erwartenden Zusammensetzung. Edman-Sequenzanalyse ergibt 2 N-terminale Aminosäurereste Leu(1) und Asn(37). Bei der Bestimmung der anticoagulatorischen Wirkung nach intravenöser Gabe am Rhesus-Affen zeigt dieses Hirudin-Derivat trotz erheblicher struktureller Änderungen überraschenderweise eine nahezu gleiche Wirksamkeit wie das Ausgangshirudin.

### Beispiel 5:

H•Thr-O-PEG₁₅₀₀ wird durch Umsetzung von Polyethylenglykolmonomethylether und tert.-Butoxycarbonyl-Threonin, z.B. in einer Eintopfreaktion mit Hydroxybenzotriazol und Dicyclohexylcarbodiimid nach bekannten peptidchemischen Methoden hergestellt.

3 g gentechnisch gewonnenes Desulfato-Tyr63-Hirudin (Sequenzprotokoll SEQ ID NO: 1) werden mit 80 g H•Thr-O-PEG₁₅₀₀ wie in Beispiel 2 mit 250 mg Chymotrypsin zur Reaktion gebracht. Als Lösungsvermittler werden dem Reaktionsgemisch zusätzlich 35 ml Dimethylformamid zugesetzt.

Nach Beendigung des Reaktion wird das Reaktionsgemisch mit 200 ml Methanol verdünnt und unumgesetztes Hirudin sowie PEG-Hirudin durch Zugabe von Dimethylether gefällt.

Das abzentrifugierte Rohprodukt wird mit einem Gemisch aus Methanol/Ether (2:1, V/V) dreimal gewaschen und unter vermindertem Druck getrocknet. Die Trennung von PEG-Hirudin und Hirudin kann wie in Beispiel 2 mit Hilfe der präparativen HPLC oder besser mit Hilfe der chromatographischen Reinigung an einer Kationenaustauschersäule (Fractogel-EMD-S0₃; Merck, Darmstadt) erfolgen. Ausbeute: 1,9 g (Hiru- dinl-63)-Thr-O-PEG, siehe Sequenzprotokoll SEQ ID NO:6.

Die Aminosäureanalyse des Produktes entspricht der nach der Aminosäuresequenz zu erwartenden Zusammensetzung.

Die Anwesenheit der PEG-Konjugation wird durch eine chymotryptische Verdauung in verdünnter wäßriger Lösung und anschließendem dünnschichtchromatographischen Vergleich mit Thr-O-PEG und freiem Threonin gezeigt. Innerhalb der analytischen Grenzen kann dabei kein freies Threonin nachgewiesen werden.

### Beispiel 6:

3 g gentechnisch gewonnenes Desulfato-Tyr63-Hirudin (Sequenzprotokoll SEQ ID NO:1) werden zusammen mit 25 g D-Glu(OBut)₂-Acetat (Sigma, Deisenhofen) in 6 ml Wasser gelöst und mit 190 mg Trypsin versetzt. Der pH-Wert der Lösung wird auf 4,5 eingestellt und die Reaktionsmischung 72 Stunden bei 40 _{°} C gelagert.

Das weitere Vorgehen und die Aufarbeitung erfolgen analog zu Beispiel 2. Zur Struktur des aus zwei Peptidketten bestehenden Produktes siehe Sequenzprotokolle der Teilsequenzen SEQ ID NO:7 und SEQ ID NO: 5.

### Beispiel 7:

3 g gentechnisch gewonnenes Desulfato-Tyr63-Hirudin (Sequenzprotokoll SEQ ID NO:1) werden zusammen mit 30 g D-Glu(OBut)₂-Acetat (Sigma, Deisenhofen) in 6 ml Wasser gelöst und mit 190 mg Chymotrypsin versetzt. Der pH-Wert der Lösung wird auf 4,5 eingestellt und die Reaktionsmischung 72 Stunden bei 4 _{°} C gelagert.

Das weitere Vorgehen und die Aufarbeitung erfolgen analog zu Beispiel 2.

Die Struktur des Produktes, besteht aus einer Kette (Sequenzprotokoll SEQ ID NO: 8.

## Patentansprüche

1. Verbindung der Formel I oder II,
AO-A1-A2-(Hirudin 3-36)-(Y), (Hirudin 37-65) (I),
A0-A1-A2-(Hirudin 3-63)-(Y) (II),
A1 ist ein Aminosäurerest,
A2 ist ein Aminosäurerest,
AO ist ein Aminosäurerest oder Wasserstoffatom,
Y ist ein Aminderivat der Formel Illa oder Illb:
H₂ N-R-X (Illa),
A-R¹⁻X (Illb),
dabei ist:
A
a) ein Aminosäurerest,
b) ein Peptid mit 2 bis 10 Aminosäureresten,
R
a) (C₁-C₁₀)-Alkyl, verzweigt oder geradkettig,
b) (C₁-C₁₀)-Alkyl, verzweigt oder geradkettig, ein- oder mehrfach unabhängig voneinander substituiert durch:
1) Phenyl,
2) Indolyl,
3) Imidazolyl oder
4) Phenyl, ein- oder mehrfach substituiert durch Hydroxyl,
c) Phenyl oder
d) Naphthyl,
R
a) Wasserstoffatom,
b) kovalente Bindung,
c) Zucker wie Glucose, Fructose, Mannose, Galactose, Ribose, Ribulose oder Xylose,
d) Polysaccharid, enthaltend 2 bis 10 Zucker, oder
e) -[O-(CH₂)ₘ]ₙ-, dabei ist: m eine ganze Zahl 2, 3, 4 oder 5 und n eine ganze Zahl von 1 bis 100, und
X
a) Wasserstoffatom,
b) -O_{R2},
c) -S_{R2},
d) -NH_{R}2,
e) -COOR² oder
f) A, dabei ist:
R²
1) Wasserstoffatom,
2) (C₁-C₁₀)-Alkyl, verzweigt oder geradkettig,
3) (C₁-C₁₀)-Alkyl, verzweigt oder geradkettig, einoder mehrfach substituiert durch:
3.1 Phenyl,
3.2 Indolyl,
3.3 Imidazolyl oder
3.4 Phenyl, ein- oder mehrfach substituiert durch Hydroxyl,
4) Phenyl oder
5) Naphthyl.

2. Verbindung der Formel I oder II gemäß Anspruch 1, dabei ist:
A ein Peptid mit 2 bis 5 Aminosäureresten,
R Ethyl, substituiert durch
1) Phenyl,
2) Indolyl,
3) Imidazolyl oder
4) 4-Hydroxyphenyl,
R -[O(CH₂)ₘ]ₙ-, dabei ist:
m die Zahl 2 und
n eine ganze Zahl von 20 bis 50,
X
a) Wasserstoffatom,
b) -O_{R2},
c) -NHR² oder
d) -COO_{R}², dabei ist:
R²
1) Wasserstoffatom,
2) (C₁-C₅)-Alkyl oder
3) Phenyl.

3. Verbindung der Formel I oder II gemäß Anspruch 1 oder 2, dabei ist
A ein Peptid mit 2 bis 5 Aminosäureresten aus der Gruppe Thr oder Arg, R¹
a) Wasserstoffatom,
b) kovalente Bindung,
c) Glucose oder
d) Polyethylenglykol mit einem Molekulargewicht von 100 bis 3000 g/mol,
X
a) Wasserstoffatom oder
b) -O_{R2}, dabei ist R² tertiär Butyl.

4. Verbindung der Formel I oder II gemäß einem oder mehreren der Ansprüche 1 bis 3, dabei ist R ein Polyethylenrest mit einem Molekulargewicht von 1500 g/mol.

5. Verfahren zur Herstellung der Verbindungen gemäß den Ansprüchen 1 bis 4, dadurch gekennzeichnet, daß ein Aminderivat der Formel Illa oder Illb mit Hirudin in Gegenwart von Proteasen umgesetzt wird.

6. Verfahren gemäß Anspruch 5, dadurch gekennzeichnet, daß eine Protease aus der Gruppe Chymotrypsin, Trypsin oder trypsinähnlicher Enzyme wie Lysylendopeptidase, eingesetzt wird.

7. Arzneimittel, enthaltend eine Verbindung gemäß den Ansprüchen 1 bis 4.

8. Verwendung der Verbindung der Formel I oder II gemäß den Ansprüchen 1 bis 4 zur Herstellung eines Arzneimittels mit gerinnungshemmender Wirkung.

9. Verwendung von dem Arzneimittel gemäß Anspruch 7 zur transdermalen Applikation, insbesondere durch die lontophorese.


### SEQUENZPROTOKOLL

(1) ALGEMEINE INFORMATION:
   (i) ANMELDER:
      (A) NAME: Hoechst Aktiengesellschaft
      (B) STRASSE: -
      (C) ORT: Frankfurt am Main
      (E) LAND: Federal Republic of Germany
      (F) POSTLEITZAHL: 65926
      (G) TELEPHON: 069-305-6047
      (H) TELEFAX: 069-35-7175
   (I) TELEX: 041234-700 hod
   (ii) ANMELDETITEL: Hirudinderivate und Verfahren zu deren Herstellung
   (iii) ANZAHL DER SEQUENZEN: 8
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
         (A) LÄNGE: 65 Aminosäuren
         (B) ART: Aminosäure
         (D) TOPOLOGIE: linear
      (ii) ART DES MOLEKÜLS: Protein
      (vi) URSPRÜNLICHE HERKUNFT:
         (A) ORGANISMUS: Desulfato-Tyr63-Hirudin
      (ix) MERKMALE:
         (A) NAME/SCHLÜSSEL: Protein
         (B) LAGE: 1..65
      (xi) SEQUENZBESCHREIBUNG: SEQ ID NO: 1:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Asn Gln Cys Val Thr Gly Glu Gly Thr Pro Lys Pro 35 40 45
         Gln Ser His Asn Asp Gly Asp Phe Glu Glu Ile Pro Glu Glu Tyr Leu 50 55 60 Gln 65
   (2) INFORMATION ZU SEQ ID NO: 2:
      (i) SEQUENZ CHARAKTERISTIKA:
         (A) LÄNGE: 64 Aminosäuren
         (B) ART: Aminosäure
         (D) TOPOLOGIE: linear
      (ii) ART DES MOLEKÜLS: Protein
      (vi) URSPRÜNLICHE HERKUNFT:
         (A) ORGANISMUS: Hirudin-Derivat
      (ix) MERKMALE:
         (A) NAME/SCHLÜSSEL: Protein
         (B) LAGE: 1..64
      (xi) SEQUENZBESCHREIBUNG: SEQ ID NO: 2:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Asn Gln Cys Val Thr Gly Glu Gly Thr Pro Lys Pro 35 40 45
         Gln Ser His Asn Asp Gly Asp Phe Glu Glu Ile Pro Glu Glu Tyr Thr 50 55 60
   (2) INFORMATION ZU SEQ ID NO: 3:
      (i) SEQUENZ CHARAKTERISTIKA:
         (A) LÄNGE: 66 Aminosäuren
         (B) ART: Aminosäure
         (D) TOPOLOGIE: linear
      (ii) ART DES MOLEKÜLS: Protein
      (vi) URSPRÜNLICHE HERKUNFT:
         (A) ORGANISMUS: Hirudin-Derivat
      (ix) MERKMALE:
         (A) NAME/SCHLÜSSEL: Protein
         (B) LAGE: 1..66
      (xi) SEQUENZBESCHREIBUNG: SEQ ID NO: 3:
         Leu Thr Tyr Thr Asp Cys Thr Glu Ser Gly Gln Asn Leu Cys Leu Cys 1 5 10 15
         Glu Gly Ser Asn Val Cys Glu Gln Gly Asn Lys Cys Ile Leu Gly Ser 20 25 30
         Asp Gly Glu Lys Asn Gln Cys Val Thr Gly Glu Gly Thr Pro Lys Pro 35 40 45
         Gln Ser His Asn Asp Gly Asp Phe Glu Glu Ile Pro Glu Glu Tyr Thr 50 55 60
         Arg Arg 65
   (2) INFORMATION ZU SEQ ID NO: 4:
      (i) SEQUENZ CHARAKTERISTIKA:
         (A) LÄNGE: 37 Aminosäuren
         (B) ART: Aminosäure
         (D) TOPOLOGIE: linear
      (ii) ART DES MOLEKÜLS: Protein
      (ix) MERKMALE:
         (A) NAME/SCHLÜSSEL: Protein
         (B) LAGE: 1..37
      (xi) SEQUENZBESCHREIBUNG: SEQ ID NO: 4:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Thr 35
      (2) INFORMATION ZU SEQ ID NO: 5:
         (i) SEQUENZ CHARAKTERISTIKA:
            (A) LÄNGE: 29 Aminosäuren
            (B) ART: Aminosäure
            (D) TOPOLOGIE: linear
         (ii) ART DES MOLEKÜLS: Protein
         (ix) MERKMALE:
            (A) NAME/SCHLÜSSEL: Protein
            (B) LAGE: 1..29
         (xi) SEQUENZBESCHREIBUNG: SEQ ID NO: 5:
            Asn Gln Cys Val Thr Gly Glu Gly Thr Pro Lys Pro Gln Ser His Asn 1 5 10 15
            Asp Gly Asp Phe Glu Glu Ile Pro Glu Glu Tyr Leu Gln 20 25
   (2) INFORMATION ZU SEQ ID NO: 6:
      (i) SEQUENZ CHARAKTERISTIKA:
         (A) LÄNGE: 64 Aminosäuren
         (B) ART: Aminosäure
         (D) TOPOLOGIE: linear
      (ii) ART DES MOLEKÜLS: Protein
      (vi) URSPRÜNLICHE HERKUNFT:
         (A) ORGANISMUS: Hirudin-Derivat
      (ix) MERKMALE:
         (A) NAME/SCHLÜSSEL: Protein
         (B) LAGE: 1..64
      (xi) SEQUENZBESCHREIBUNG: SEQ ID NO: 6:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Asn Gln Cys Val Thr Gly Glu Gly Thr Pro Lys Pro 35 40 45
         Gln Ser His Asn Asp Gly Asp Phe Glu Glu Ile Pro Glu Glu Tyr Xaa 50 55 60
   (2) INFORMATION ZU SEQ ID NO: 7:
      (i) SEQUENZ CHARAKTERISTIKA:
         (A) LÄNGE: 37 Aminosäuren
         (B) ART: Aminosäure
         (D) TOPOLOGIE: linear
      (ii) ART DES MOLEKÜLS: Protein
      (ix) MERKMALE:
         (A) NAME/SCHLÜSSEL: Protein
         (B) LAGE: 1..37
      (xi) SEQUENZBESCHREIBUNG: SEQ ID NO: 7:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Xaa 35
   (2) INFORMATION ZU SEQ ID NO: 8:
      (i) SEQUENZ CHARAKTERISTIKA:
         (A) LÄNGE: 64 Aminosäuren
         (B) ART: Aminosäure
         (D) TOPOLOGIE: linear
      (ii) ART DES MOLEKÜLS: Protein
      (vi) URSPRÜNLICHE HERKUNFT:
         (A) ORGANISMUS: Hirudin-Derivat
      (ix) MERKMALE:
         (A) NAME/SCHLÜSSEL: Protein
         (B) LAGE: 1..64
      (xi) SEQUENZBESCHREIBUNG: SEQ ID NO: 8:
         Leu Thr Tyr Thr Asp Cys Thr Glu Ser Gly Gln Asn Leu Cys Leu Cys 1 5 10 15
         Glu Gly Ser Asn Val Cys Gly Gln Gly Asn Lys Cys Ile Leu Gly Ser 20 25 30
         Asp Gly Glu Lys Asn Gln Cys Val Thr Gly Glu Gly Thr Pro Lys Pro 35 40 45
         Gln Ser His Asn Asp Gly Asp Phe Glu Glu Ile Pro Glu Glu Tyr Xaa 50 55 60